(19) Europäisches Patentamt
European Patent Office
Office européen des brevets

(11) Veröffentlichungsnummer: **0 000 494**
**B1**

(12) # EUROPÄISCHE PATENTSCHRIFT

(21) Anmeldenummer: **78100355.3**

(22) Anmeldetag: **11.07.78**

(51) Int. Cl.³: **C 07 H 13/04,** D 06 P 1/642, D 06 P 1/649, C 11 D 1/38, C 07 C 103/46, C 07 C 103/84, C 07 C 125/06, C 07 C 127/15, C 07 C 127/17, C 07 C 127/19

(54) **Ester von acylierten Aminocarbonsäuren, Verfahren zu ihrer Herstellung und ihre Verwendung beim Färben von Textilmaterialien**

(30) Priorität: **19.07.77 DE 2732557**

(43) Veröffentlichungstag der Anmeldung: **07.02.79 Patentblatt 79/03**

(45) Bekanntmachung des Hinweises auf die Patenterteilung: **15.10.80 Patentblatt 80/21**

(84) Benannte Vertragsstaaten: **CH DE FR GB**

(56) Entgegenhaltungen:
**BE - A - 672 897**
**DE - A - 2 349 278**
**FR - A - 1 337 215**
**FR - A - 2 203 806**
**NL - A - 275 949**
**NL - A - 295 975**

(73) Patentinhaber: **BAYER AG**
**Zentralbereich Patente, Marken und Lizenzen**
**D - 5090 Leverkusen 1**
**Bayerwerk (DE)**

(72) Erfinder: **Walz, Klaus, Dr.**
**Domblick 4**
**D - 5090 Leverkusen 31 (DE)**
**Tamer, Ergun, Dipl.-Ing.**
**Zedernweg 37**
**D - 5090 Leverkusen 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 494 B1

Courier Press, Leamington Spa, England.

Ester von acylierten Aminocarbonsäuren, Verfahren zu ihrer Herstellung und ihre Verwendung beim Färben von Textilmaterialien

Gegenstand der Erfindung sind oberflächenaktive Ester von Acylaminocarbonsäuren und Polyolen. Sie sind dadurch gekennzeichnet, daß sie sich von Acylaminocarbonsäuren der Formel

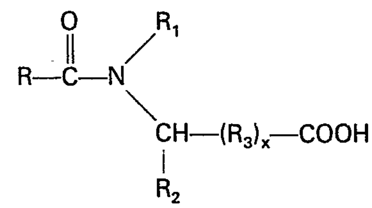

I

in der

R für Wasserstoff, $C_1$—$C_4$-Alkyl, $C_1$—$C_{22}$-Alkoxy, $C_3$—$C_{22}$-Alkenyloxy, Phenyl, Naphthyl, Benzyl, Phenylethyl, Naphthylmethyl, Phenyloxy, Benzyloxy, Phenylethyloxy oder für einen Rest

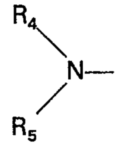 steht,

$R_1$, $R_4$ und $R_5$ für Wasserstoff, $C_1$—$C_{22}$-Alkyl, $C_3$—$C_{22}$-Alkenyl, Cyclohexyl, Tetrahydronaphthyl, Dekahydronaphthyl, Abiethyl, Phenyl, Naphthyl, Benzyl oder Phenylethyl stehen, oder

$R_5$ für einen Rest der Formel

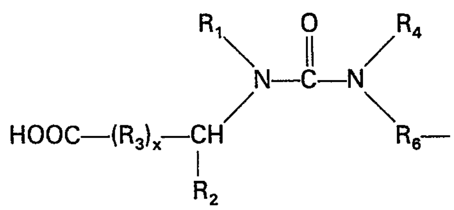 steht,

$R_6$ für $C_2$—$C_8$-Alkylen oder einen Rest der Formeln

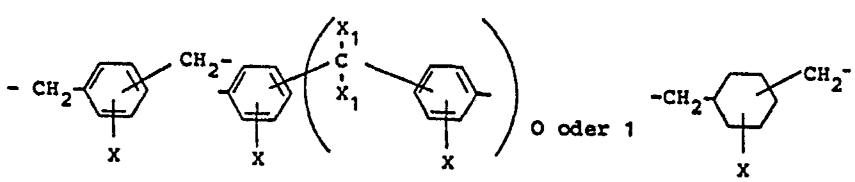

0 oder 1

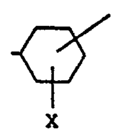 X, $X_1$ = Wasserstoff, $C_1$—$C_4$-Alkyl,

$R_2$ für H, COOH, COO $C_1$—$C_4$-Alkyl oder $C_1$—$C_6$-Alkyl,
$R_3$ für $C_1$—$C_{10}$-Alkylen und
x für 0 oder 1 stehen,

und in der mindestens einer der Reste $R_1$, $R_2$, $R_3$, $R_4$ und $R_5$ mindestens 6 Kohlenstoffatome enthält, und die Alkyl-, Alkenyl- und die cyclischen Reste durch Fluor, Chlor, Brom, Cyan, Carboxyl, $C_1$—$C_4$-Alkoxycarbonyl, Carbamoyl oder $C_1$—$C_4$-Alkoxy und die cyclischen Reste außerdem durch $C_1$—$C_4$-Alkyl substituiert sein können, und von Glycerin, Erythrit, Erythrose, Pentosen oder Hexosen, Pentiten oder Hexiten, Di- oder Oligosacchariden, Hydrolysate von Polysacchariden, Polysacchariden, Formosen, Polyvinylalkoholen sowie Hydroxyalkylethern oder partiellen Estern oder Ethern von Polyhydroxylverbindungen ableiten.

Gegenstand der Erfindung sind außerdem ein Verfahren zu ihrer Herstellung sowie ihre Verwendung als Hilfsmittel beim Färben von Textilmaterialien.

Die obenflächenaktiven Ester können in an sich bekannter Weise hergestellt werden, indem Aminocarbonsäuren der Formel

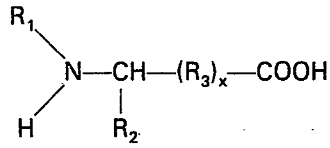

(II)

wobei

$R_1$, $R_2$, $R_3$, und x die in Formel (I) genannte Bedeutung besitzen,

ihre Alkylester, Anhydride oder Halogenide mit den vorstehend genannten Polyolen und mit Acylierungsmitteln der Formeln

$$R\text{—}CO\text{—}Y \quad R_4\text{—}NCO \text{ oder } OCN\text{—}R_6\text{—}NCO$$

worin

$R$, $R_4$ und $R_6$ die bei Formel (I) angegebene Bedeutung haben, und

Y für eine $C_1$—$C_4$-Alkoxygruppe, Halogen oder die Gruppe —O—COR steht,

in beliebiger Reihenfolge umgesetzt werden.

Die Acylierung kann in Gegenwart von Katalysatoren oder molaren Mengen an Säureakzeptoren durchgeführt werden.

Die Verfahrensbedingungen können in weiten Grenzen variieren. Durch die Wahl des Molverhältnisses zwischen Aminocarbonsäure oder Acylaminocarbonsäure und Polyol lassen sich die Eigenschaften der hergestellten Ester variieren.

Im allgemeinen werden pro Äquivalent Carboxylgruppe 0,5—2,0, vorzugsweise 0,8—1,2 Mol Polyol eingesetzt.

Die Reaktion der Acylaminocarbonsäuren der Formel (I) mit den Polyolen kann mit oder ohne Zusatz von Lösungsmitteln bei Temperaturen von 20—180°C, vorzugsweise bei 70—140°C durchgeführt werden. Im Falle der bevorzugt eingesetzten niedrigen Alkylester der Acylaminocarbonsäuren können übliche Lösungsmittel wie Xylol, Dimethylformamid, Dimethylsulfoxid, N-Methylpyrrolidin oder Dimethylacetamid verwendet werden. Es empfiehlt sich dabei, saure oder insbesondere basische Katalysatoren in Mengen von 0,5—20% (bezogen auf Carbonsäure) dem Reaktionsgemisch zuzugeben. Geeignete saure Katalysatoren sind beispielsweise Chlorwasserstoff, Schwefelsäure, Toluolsulfonsäure oder saure Ionenaustauscher. Als basische Katalysatoren können Alkali- oder Erdalkalihydroxide, -carbonate oder -alkoholate, sowie organische Basen verwendet werden.

Die Acylierungsreaktion wird bei 0 bis 120°C, vorzugsweise bei 20 bis 90°C, gegebenenfalls in Lösungsmitteln, wie niederen Alkoholen, Glykolen, Ketonen, aromatischen Kohlenwasserstoffen, Carbonsäureestern oder -amiden durchgeführt.

Bevorzugte erfindungsgemäße Ester werden hergestellt, indem man niedrige Alkylester, insbesondere Methylester, von Acylaminocarbonsäuren der Formel

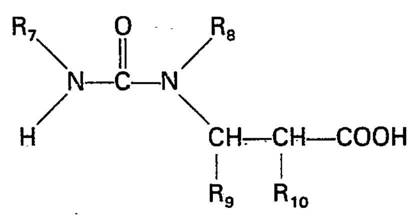

(III)

oder

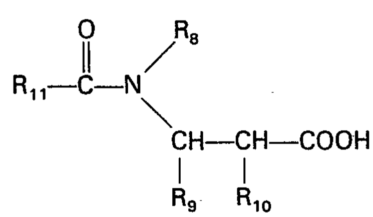

(IV)

worin

$R_7$ für $C_1$—$C_{22}$-Alkyl, $C_3$—$C_{22}$-Alkenyl, Cyclohexyl, Tetrahydronaphthyl, Dekahydronaphthyl, Abietyl, Phenyl, Naphthyl, Benzyl, Phenyläthyl oder einen Rest der Formel

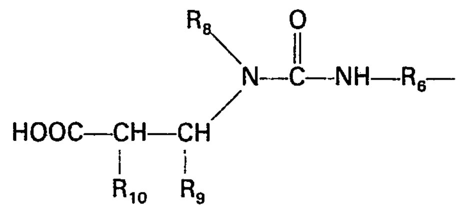

wobei

$R_6$ die in Formel (I) angegebene Bedeutung hat,

$R_8$ für Wasserstoff, $C_1$—$C_{22}$-Alkyl, $C_3$—$C_{22}$-Alkenyl, Cyclohexyl, Tetrahydronaphthyl, Dekahydronaphthyl, Abietyl, Benzyl oder Phenyläthyl,

$R_9$ für Wasserstoff oder Carboxyl und

$R_{10}$ und $R_{11}$ für Wasserstoff oder Methyl stehen, mindestens einer der Reste $R_7$ oder $R_8$ mindestens 8 Kohlenstoffatome besitzt, und die Alkyl-, Alkenyl- und cyclischen Reste die in Formel (I) genannten Substituenten tragen können,

mit mindestens 6 Kohlenstoffatome enthaltenden aliphatischen Polyolen umsetzt.

Bevorzugt werden dabei Sorbit, Glucose, Alkyl- oder Hydroxyalkylglycoside, Trehalose, Raffinose, Saccharose oder deren Mischungen und insbesondere Saccharose verwendet.

Die bevorzugten Ester lassen sich auch herstellen, indem Ester aus Aminocarbonsäuren der Formel

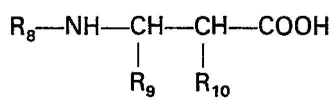 (V)

und mindestens 6 Kohlenstoffatome enthaltenden Polyolen mit Isocyanaten der Formel

$$R_7\text{—}N=C=O$$

wobei $R_7$—$R_{10}$ die obengenannte Bedeutung haben,

oder mit Ameisensäuremethylester, Acetylchlorid oder Essigsäureanhydrid umgesetzt werden. Als Isocyanate seien beispielsweise genannt: Methyl-, Äthyl-, Propyl-, Butyl-, Hexyl-, 6-Chlorhexyl-, Octyl-, Decyl-, Dodecyl-, Hexadecyl-, Octadecyl-, Cyclohexyl-, Phenyl-, Naphthyl-, Allylisocyanat, sowie Butylen-, Hexamethylen-, Phenylen-, Toluylen-, Hexahydrophenylendiisocyanat.

Als Aminocarbonsäureester werden vorzugsweise solche Ester verwendet, denen als Aminocarbonsäuren solche der Formel

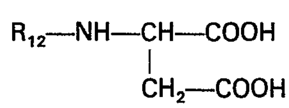 (VI) oder

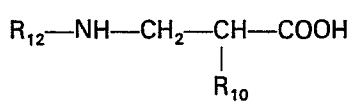 (VII)

wobei

$R_{10}$ die obengenannte Bedeutung hat und

$R_{12}$ für $C_1$—$C_{22}$-Alkyl oder $C_3$—$C_{22}$-Alkenyl steht,

und als aliphatische Polyole Sorbit, Glucose, Alkyl- oder Hydroxyalkylglycoside, Trehalose, Raffinose oder insbesondere Saccharose zugrunde liegen.

Besonders bevorzugte erfindungsgemäße Ester sind Ester aus Acylaminocarbonsäuren der Formeln

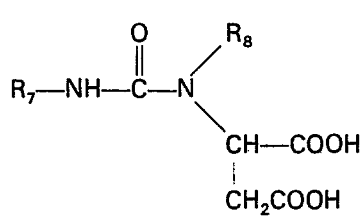 (VIII)

und

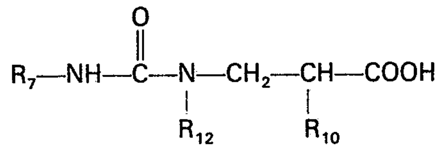

(IX)

und Saccharose, wobei $R_7$, $R_8$, $R_{10}$ und $R_{12}$ die obengenannte Bedeutung besitzen.

Als Carbonsäuren der Formel (I) eignen sich beispielsweise:

| R | $R_1$ | $R_2$ | $R_3$ | x |
|---|---|---|---|---|
| H | $C_{12}H_{25}$ | H | $CH_2$ | 1 |
| $CH_3$ | $C_{12}H_{25}$ | H | $CH_2$ | 1 |
| $CH_3$ | $C_{12}H_{25}$ | H | $CH(CH_3)$ | 1 |
| $CH_3$ | $C_{16}H_{33}$ | H | $CH_2$ | 1 |
| $CH_3$ | $C_{16}H_{33}$ | COOH | $CH_2$ | 1 |
| 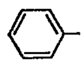 | $C_{12}H_{25}$ | COOH | $CH_2$ | 1 |
| 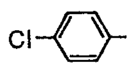 | $C_{10}H_{21}$ | H | $CH(CH_3)$ | 1 |
| $CH_3$–O | $C_{14}H_{29}$ | H | $CH_2$ | 1 |
| $C_4H_9$–O | $C_{12}H_{25}$ | H | – | 0 |
| $C_{10}H_{21}$–O | 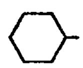 | COOH | $CH_2$ | 1 |
| $C_{18}H_{35}$–O | $CH_3$ | COOH | $CH_2$ | 1 |
| $ClCH_2CH_2$–O | $C_{18}H_{37}$ | COOH | $CH_2$ | 1 |
| $CH_3$–O | Abietyl | H | $CH(CH_3)$ | 1 |
| 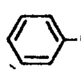-$CH_2$–O | $C_{12}H_{25}$ | COOH | $CH_2$–$CH_2$ | 1 |
| $CH_3$–NH | $C_{12}H_{25}$ | COOH | $CH_2$ | 1 |
| $CH_3$–NH | $C_{16}H_{33}$ | H | $CH_2$ | 1 |
| $CH_3$–NH | $C_{18}H_{37}$ | H | $CH(CH_3)$ | 1 |
| $CH_3$–NH | Abietyl | COOH | $CH_2$ | 1 |
| $CH_3$–NH | $C_{12}H_{25}$ | n-$C_6H_{13}$ | – | 0 |
| n-$C_4H_9$–NH | $C_{12}H_{25}$ | COOH | $CH_2$ | 1 |
| n-$C_4H_9$–NH | $C_{14}H_{29}$ | COOH | $CH_2$ | 1 |
| iso-$C_4H_9$–NH | $C_{18}H_{35}$ | COOH | $CH_2$ | 1 |
| iso-$C_4H_9$–NH | $C_{18}H_{37}$ | H | $(CH_2)_3$ | 1 |
| Cl–$(CH_2)_6$–NH | $C_{10}H_{21}$ | COOH | $CH_2$ | 1 |
| Cl–$(CH_2)_6$–NH | Abietyl | COOH | $CH_2$ | 1 |

| R | $R_1$ | $R_2$ | $R_3$ | x |
|---|---|---|---|---|
| 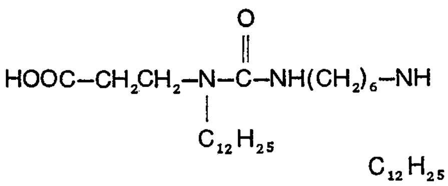 | $C_{12}H_{25}$ | H | $CH_2$ | 1 |
| $C_{12}H_{25}-NH$ | $CH_3$ | COOH | $CH_2$ | 1 |
| $C_{12}H_{25}-NH$ | $CH_2=CH-CH_2$ | COOH | $CH_2$ | 1 |
| $C_{12}H_{25}-NH$ | 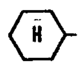 | H | $CH_2$ | 1 |
| $C_{12}H_{25}-NH$ | 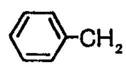 | COOH | $CH_2$ | 1 |
| $C_{12}H_{25}-NH$ | H | H | $(CH_2)_4$ | 1 |
| $C_{12}H_{25}-NH$ | $C_{12}H_{25}$ | COOH | $CH_2$ | 1 |
| $C_{14}H_{29}-NH$ | $CH_3$ | COOH | $CH_2$ | 1 |
| $C_{14}H_{25}$ NH | 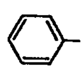 | H | $CH_2$ | 1 |
| $C_{14}H_{29}NH$ | 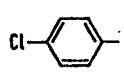 | H | – | 0 |
| $C_{16}H_{33}NH$ | 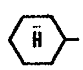 | COOH | $CH_2$ | 1 |
| $C_{18}H_{37}NH$ | $C_4H_9$ | COOH | $CH_2$ | 1 |
| $C_{18}H_{37}-NH$ | $CH_3$ | $COOCH_3$ | $CH_2$ | 1 |
| 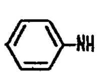 | $C_8H_{17}$ | COOH | $CH_2$ | 1 |
| 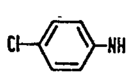 | H | H | $(CH_2)_9$ | 1 |
| 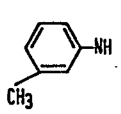 | $C_{12}H_{25}$ | H | $CH(CH_3)$ | 1 |

| R | $R_1$ | $R_2$ | $R_3$ | x |
|---|---|---|---|---|
| 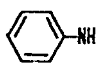 | $C_{12}H_{25}$ | COOH | $CH_2$ | 1 |
| 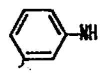 | $C_{16}H_{33}$ | COOH | $CH_2$ | 1 |
| 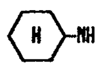 | $C_{18}H_{37}$ | COOH | $CH_2$ | 1 |
| 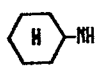 | $C_{18}H_{37}$ | H | – | 0 |
| Abietyl–NH | $CH_3$ | COOH | $CH_2$ | 1 |
| 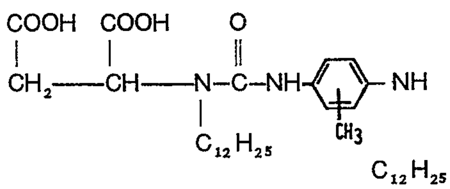 | $C_{12}H_{25}$ | COOH | $CH_2$ | 1 |
| 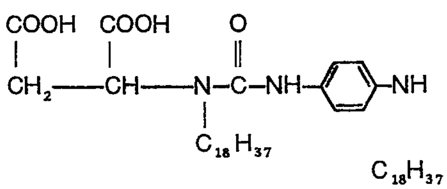 | $C_{18}H_{37}$ | COOH | $CH_2$ | 1 |
| 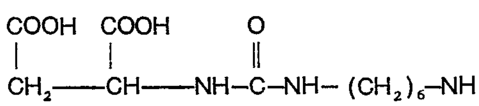 | $C_{18}H_{37}$ | COOH | $CH_2$ | 1 |
| 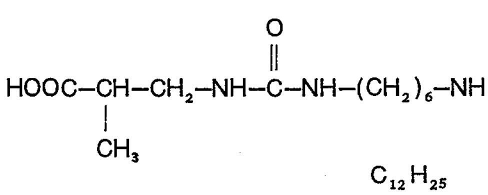 | $C_{12}H_{25}$ | $CH_3$ | – | 0 |

Die neuen Verbindungen sind biologisch voll abbaubare oberflächenaktive Verbindungen, deren Eigenschaften durch Wahl der Ausgangsmaterialien und der Mengenverhältnisse in weiten Grenzen variiert werden können. Die Verbindungen sind daher für die unterschiedlichsten Anwendungsbereiche einsetzbar, beispielsweise als Wasch-, Reinigungs- oder Netzmittel, als Emulgier-, Dispergier- oder Verdickungsmittel, sowie als Färberei- und Druckereihilfsmittel.

In bevorzugter Weise eignen sich die neuen Verbindungen zum kontinuierlichen Färben bzw. Bedrucken von Fasermaterialien mit für derartige Fasern gebräuchlichen Farbstoffen. Das Verfahren besteht darin, daß man die Fasermaterialien mit einer wäßrigen Flotte imprägniert bzw. bedruckt, die die Farbstoffe und die erfindungsgemäßen Verbindungen enthält, und anschließend einer thermischen Nachbehandlung unterwirft.

Die erforderlichen Mengen an den erfindungsgemäßen Produkten lassen sich durch Vorversuche leicht ermitteln; im allgemeinen erweisen sich die Mengen von etwa 5—20 g pro Liter Foulardierflotten als ausreichend.

Als Farbstoffe kommen für das erfindungsgemäße Verfahren alle Farbstoffe in Betracht, die üblicherweise für das Färben und Bedrucken von Textilmaterialien verwendet werden, z.B. Säurefarbstoffe, Reaktivfarbstoffe, Nachchromierungsfarbstoffe, 1:1- sowie 1:2-Metallkomplexfarbstoffe, kationische Farbstoffe, Substantivfarbstoffe und Dispersionsfarbstoffe.

Beispielsweise sind folgende Farbstoffe des Colour Index, 3. Edition, Vol 5, geeignet:

Als saure Farbstoffe:

C.I. Acid Yellow 79
C.I. Acid Red 80
C.I. Acid Green 44
C.I. Acid Red 111

Als Nachchromierungsfarbstoffe:

C.I. Mordant Yellow 5
C.I. Mordant Black 65
C.I. Mordant Brown 21

Als 1:1-Metallkomplexfarbstoffe:

C.I. Acid Yellow 54
C.I. Acid Red 214

Als 1:2-Metallkomplexfarbstoffe:

C.I. Acid Blue 199
C.I. Acid Red 277
C.I. Acid Brown 253
C.I. Acid Blue 151
C.I. Acid Green 86

Als Reaktivfarbstoffe:

C.I. Reactive Blue 94
C.I. Reactive Red 100
C.I. Reactive Yellow 69

Als kationische Farbstoffe:

C.I. Basic Yellow 11
C.I. Basic Blue 3
C.I. Basic Red 18

Als Substantivfarbstoffe:

C.I. Direct Orange 46
C.I. Direct Blue 84

Als Dispersionsfarbstoffe:

C.I. Disperse Yellow 5
C.I. Disperse Yellow 60
C.I. Disperse Blue 56
C.I. Disperse Blue 73

Das Färben und Bedrucken kann z.B. auf folgende Weise vorgenommen werden:

Die Farbstoffe werden unter kräftigem Rühren, gegebenenfalls unter Erwärmen auf etwa 70—80°C, im Wasser verteilt; dann werden die erfindungsgemäß zu verwendenden Verbindungen zugesetzt und die erhaltene Mischung gegebenenfalls mit niederen aliphatischen Carbonsäuren, wie Essigsäure, Ameisensäure oder Oxalsäure, angesäuert oder mit in der Hitze Säure abspaltenden Verbindungen, wie Ammoniumsulfat oder Ammoniumacetat, versetzt. Mit dieser Flotte werden die Fasermaterialien z.B. auf einem Foulard imprägniert und anschließend einer thermischen Nachbehandlung unterworfen. Diese kann insbesondere bei Wolle und Polyacrylnitrilfasern in einer Behandlung bei etwa 100—110°C bestehen oder insbesondere bei synthetischen Polyamidfasern und Polyesterfasern in einer Trockenhitze-behandlung bei Temperaturen von 120 bis 220°C. Nach der thermischen Behandlung wird das Fasermaterial gewaschen, gespült und getrocknet.

Die Färbungen und Drucke lassen sich durch Mitverwendung von Harnstoff, Thioharnstoff oder organischen Lösungsmitteln, die ein gutes Lösevermögen für den jeweils benutzten Farbstoff besitzen, in vielen Fällen noch verbessern; genannt seien in diesem Zusammenhang z.B. Butanol, Hexanol, 2-Äthylhexanol, Cyclohexanol, Benzylalkohol, Diäthylenglykol, Triäthylenglykol, Thiodiäthylenglykol, der Monoäthyläther und Monobutyläther des Äthylenglykols oder des Diäthylenglykols.

Eine Verbesserung der Färbung oder des Druckes läßt sich in vielen Fällen auch durch eine Mitverwendung von Alkylsulfonamiden und/oder anionaktiven Verbindungen erzielen, z.B. Alkylsulfate, Alkylarylsulfonate, Fettsäuresalze oder vorzugsweis Alkylsulfonate, die als Natrium-, Kalium-, Ammonium- oder auch als Mono-, Di- oder Triäthanolaminsalze eingesetzt werden können.

Es empfiehlt sich ferner, zur Verbesserung der Gleichmäßigkeit der Färbung ein säurebeständiges Verdickungsmittel zu den Foulardierflotten zuzusetzen, beispielsweise ein Johannisbrotkornmehlderivat.

Mit Hilfe des erfindungsgemäßen Verfahren gelingt es, Textilmaterialien, beispielsweise Flocken, Spinnkabel, Kammzüge, Gewebe, Gewirke oder Vliesen z.B. aus natürlichen und synthetischen Polyamiden, Polyester, Polyacrylnitril, sowie Cellulosefasern, sowie deren Mischungen untereinander zu färben oder zu bedrucken.

Ein besonderer Vorteil des erfindungsgemäßen Verfahrens besteht darin, daß seine Anwendung universell ist, d.h. alle färbbaren Fasermaterialien nach dem Verfahren gefärbt werden können.

Die neuen Verbindungen ermöglichen eine ausgezeichnete Fixierung der Farbstoffe. Die erhaltenen Färbungen zeichnen sich durch gute Naß-, Schweiß- und Reibechtheiten aus.

Das Auftreten eines Grauschleiers wird durch die neuen Verbindungen verhindert.

Oberflächenaktive Derivate von Polyhydroxy-Verbindungen, die frei von N-acylierten Aminocarbonsäure-Gruppen sind, sind aus der FR - A - 1 337 215 und der DE - A - 2 349 278 bekannt. Bei den Verbindungen der französischen Patentschrift handelt es sich um Ester aus gegebenenfalls durch OH substituierten Carbonsäuren und Polyhydroxyverbindungen, deren Wirksamkeit in der Färberei den erfindungsgemäßen Verbindungen unterlegen ist. Die Verbindungen der deutschen Offenlegungsschrift stellen Polyhydroxyalkyl-harnstoff-Derivate dar, die eine freie Aminogruppe tragen und frei von Carbonsäureester-Gruppen sind. Sie bilden mit Säure- und Metallkomplex-Farbstoffen Ausfällungen und sind darum in entsprechenden Färbebädern nicht anwendbar.

Aus der BE - A 672 897 bzw. der NL - A - 295 975 sind Verfahren zum Färben von Polyestern bzw. Wolle und synthetischen Polyamiden unter Verwendung von N-Acylaminocarbonsäure(salze)n, die frei von Polyhydroxyalkyl-Gruppen sind, bekannt. Die Verfahren besitzen den Nachteil, daß sie eine genaue pH-Einstellung erforderlich machen. Geringfügig höhere Säurekonzentrationen führen zum Ausölen der Koazervat-Phase, während bei geringfügig niedrigeren Säurekonzentrationen die Gefahr der Verkochung empfindlicher Dispersionsfarbstoffe besteht. Beim Einsatz von Chromierungsfarbstoffen sind die Verbindungen wegen der Bildung unlöslicher Chromseifen ungeeignet.

Ester von N-Acylaminocarbonsäuren und gegebenenfalls oxalkylierten Alkoholen mit emulgierender Wirkung sind aus der FR - A - 2 203 806 bekannt. Sie sind im Gegensatz zu den erfindungsgemäßen Verbindungen nicht zur Herstellung stabiler wäßriger Färbeflotten geeignet. Mit den Färbeflotten werden unegale Färbungen erhalten.

Ein Färbeverfahren in Gegenwart von Polyethern und Amiden höhermolekularer Carbonsäuren ist aus der Ne - A - 275 949 bekannt. Nach diesem Verfahren kann nicht vorteilhaft mit Metallkomplexfarbstoffen gefärbt werden. Die zurückhaltende Wirkung der Hilfsmittel führt zu schlechten Farbausbeuten und zum Ausbluten der Färbungen bei der Wäsche nach dem Dämpfen.

Die in den nachfolgenden Beispielen angegebenen Teile sind Gewichtsteile.

BEISPIEL 1

Aus einer Lösung von 136,8 Teilen Saccharose in 600 Teilen Dimethylformamid werden bei 90—100°C im Vakuum 50 ml Dimethylformamid innerhalb von 1—2 Stunden über eine Kolonne abdestilliert. Anschließend werden 4 Teile Kaliumcarbonat und 94 Teile eines Asparaginsäuredimethylesters der Formel

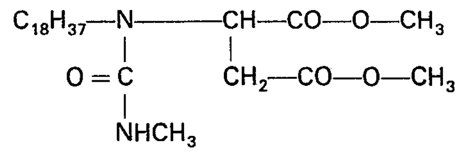

der durch Umsetzung von N-Octadecylasparaginsäure-dimethylester mit Methylisocyanat hergestellt wurde, zugesetzt. Aus der Reaktionsmischung werden dann unter Rühren innerhalb von 6 Stunden bei 90—100°C und ca. 110 Torr etwa 100 Teile einer Mischung aus Methanol und Dimethylformamid abdestilliert. Der Kolbenrückstand wird bei 100°C und einem Vakuum bis 2 Torr von Dimethylformamid befreit. Es werden 237 Teile eines braunen Harzes erhalten, das in der Kälte zu einem spöden, pulverisierbaren Harz erstarrt. Das Produkt is klar wasserlöslich, schmilzt bei 150—160°C, besitzt eine OH-Zahl von 610, eine Verseifungszahl von 72 und erniedrigt die Oberflächenspannung des Wassers in 0,1%iger Lösung auf 46 dyn/cm.

BEISPIEL 2

Unter den in Beispiel 1 angegebenen Bedingungen wird eine Lösung von 136,8 Teilen Saccharose in 600 Teilen Dimethylformamid in Gegenwart von 2 Teilen Kaliumcarbonat mit 77,2 Teilen eines Asparaginsäureesters der Formel

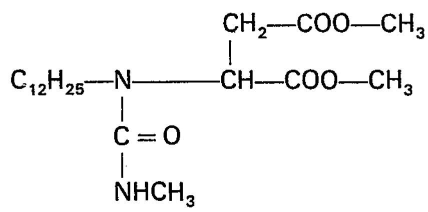

umgesetzt, der durch Umsetzung von N-Dodecylasparaginsäuredimethylester mit Methylisocyanat hergestellt wurde. Nach dem Abdestillieren des Dimethylformamids werden 200 Teile eines bei Raumtemperatur spröden, pulverisierbaren Harzes erhalten. Das klar wasserlösliche Produkt besitzt eine OH-Zahl von 650, eine Verseifungszahl von 78 und erniedrigt die Oberflächenspannung des Wassers in 0,1%iger Lösung auf 35 dyn/cm.

BEISPIEL 3

Unter den in Beispiel 1 angegebenen Bedingungen wird eine Lösung von 171 Teilen Saccharose in 650 Teilen Dimethylformamid in Gegenwart von 4 Teilen Kaliumcarbonat mit 108,8 Teilen eines Asparaginsäureesters der Formel

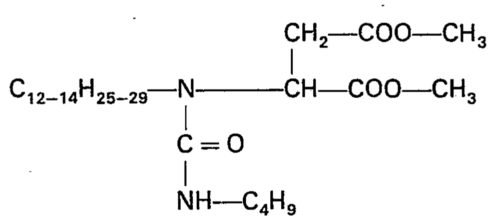

umgesetzt, der durch Umsetzung von N-Kokosfettalkylasparaginsäure-dimethylester mit n-Butylisocyanat hergestellt wurde. Nach Abdestillieren des Dimethylformamids werden 278 Teilen eines klar wasserlöslichen, harzartigen Produktes erhalten.

Das Produkt besitzt eine OH-Zahl von 540, eine Verseifungszahl von 60 und erniedrigt die Oberflächenspannung des Wassers in 0,1%iger Lösung auf 33 dyn/cm.

BEISPIEL 4

In der in Beispiel 1 beschriebenen Weise werden 136,8 Teile Saccharose und 2 Teile Kaliumcarbonat in 600 Teilen Dimethylformamid mit 100 Teilen eines Asparaginsäureesters der Formel

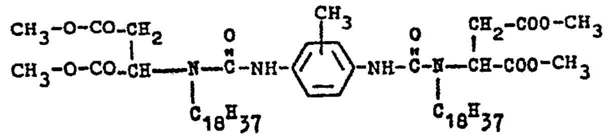

umgesetzt, der durch Umsetzung von 207 Teilen N-Octadecylasparaginsäure-dimethylester mit 30 Teilen Toluylendiisocyanat erhalten wurde. Nach dem Entfernen des Dimethylformamids im Vakuum werden 220 Teile eines wasserlöslichen, spröden, pulverisierbaren Harzes erhalten.

Das Produkt besitzt eine OH-Zahl von 684 und eine Verseifungszahl von 98,5 und erniedrigt die Oberflächenspannung des Wassers in 0,1%iger Lösung auf 52 dyn/cm.

BEISPIEL 5

In der in Beispiel 1 beschriebenen Weise werden 232 Teile Saccharose und 2 Teile Kaliumcarbonat in 630 Teilen Dimethylformamid mit 152 Teilen eines Asparaginsäureesters der Formel

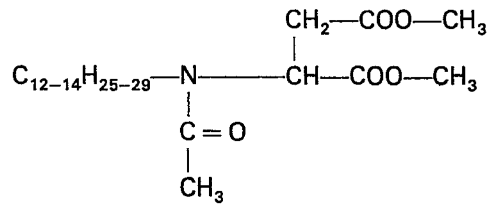

umgesetzt, der durch Umsetzung von 168 Teilen N-Kokosalkyl-asparaginsäuredimethylester mit 66 Teilen Essigsäureanhydrid hergestellt wurde. Nach dem Entfernen des Dimethylformamids im Vakuum werden 350 Teile eines harzartig erstarrenden, klar wasserlöslichen Produktes erhalten

Es schmilzt bei 80—84°C, besitzt eine OH-Zahl von 675, eine Verseifungszahl von 151 und setzt die Oberflächenspannung des Wassers in 0.1%iger Lösung auf 30,5 dyn/cm herab.

BEISPIEL 6

In der in Beispiel 1 beschriebenen Weise werden 137 Teile Saccharose und 2 Teile Kaliumcarbonat in 450 Teilen Dimethylformamid mit 110 Teilen eines Umsetzungsproduktes der Formel

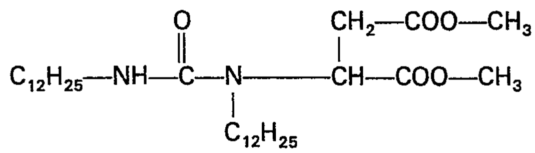

aus 255 Teilen N-Dodecyl-asparaginsäuredimethylester und 152 Teilen n-Dodecyl-isocyanat umgesetzt. Nach dem Entfernen des Lösungsmittels im Vakuum werden 229 Teile eines in Wasser leicht löslichen, harzartigen, pulverisierbaren Produktes erhalten. Es schmilzt bei 140—145°C und besitzt eine Verseifungszahl von 65 und eine Hydroxylzahl von 660.

BEISPIEL 7

In der in Beispiel 1 angegebenen Weise werden 146 Teile Sorbit und 4 g Kaliumcarbonat in 550 Teilen Dimethylformamid mit 160 Teilen eines Umsetzungsproduktes aus 1 Mol N-Kokosalkylasparaginsäure-dimethylester und 1 Mol Methylisocyanat umgesetzt. Nach dem Abdestillieren des Dimethylformamids werden 286 Teile eines klar wasserlöslichen Harzes erhalten, das zu einer klebrigen Masse erstarrt. Das Produkt besitzt eine Verseifungszahl von 85 und eine Hydroxylzahl von 826.

BEISPIEL 8

In der in Beispiel 1 angegebenen Weise werden 103 Teile Saccharose und 2 Teile Natriummethylat in 500 Teilen Dimethylformamid mit 110 Teilen der Verbindung der Formel

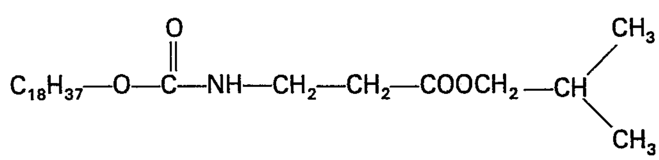

die durch Umsetzung von 3-Isocyanatopropionsäure-isobutylester mit Octadecylalkohol hergestellt wurde, umgesetzt.

An Stelle des Methanol-Dimethylformamid-Gemisches wird eine Mischung aus Isobutanol und

Dimethylformamid im Vakuum destilliert. Nach dem Entfernen des Lösungsmittels werden 195 Teile eines hellbraunen, pulverisierbaren Harzes erhalten. Das Produkt ist in warmem Wasser löslich und setzt die Oberflächenspannung des Wassers in 0,1%iger Lösung auf 55 dyn/cm herab. Die Verseifungszahl beträgt 54, die Hydroxylzahl 630.

### BEISPIEL 9

In der in Beispiel 1 angegebenen Weise werden 103 Teile Saccharose und 4 Teile Kaliumcarbonat in 500 Teilen Dimethylformamid mit 103 Teilen der Verbindung der Formel

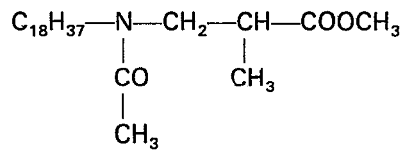

die durch Umsetzung von 3-Octadecylamino-isobuttersäure mit Essigsäureanhydrid erhalten wurde, umgesetzt. Nach dem Entfernen des Lösungsmittels im Vakuum werden 198 Teile eines wasserlöslichen, braunen Harzes erhalten. Das Produkt setzt die Oberflächenspannung des Wassers in 0,1%iger Lösung auf 39,3 dyn/cm herab. Es besitzt eine Hydroxylzahl von 552 und eine Verseifungszahl von 55.

### BEISPIEL 10

20 Teile des Farbstoffes C.I. Nr. 61590 (3. Auflage, Band 4) werden in 600 Teilen weichem Wasser von ca. 80°C gelöst; anschließend werden 8 Teile der Verbindung des Beispiels 5 und 2 Teile Natriumlaurylsulfonat unter Rühren hinzugefügt. Die Lösung wird unter ständigem Rühren zu 200 Teilen einer 4%igen wäßrigen Zubereitung eines handelsüblichen Johannisbrotkernmehlderivates hinzugegeben. Hierauf wird die homogene Lösung mit 20 Teilen Essigsäure (60%) versetzt. Abschließend wird die Farbstoffzubereitung mit kaltem, weichem Wasser auf 1000 Teile aufgefüllt.

Ein Wollkammzug wird mit dieser Farbflotte auf einem Foulard imprägniert; dabei beträgt die Flottenaufnahme etwa 100%. Anschließend wird er in einem Dämpfer mit Sattdampf bei 102—103°C während 15 Min. kontinuierlich gedämpft. Danach wird der Kammzug auf einer Lisseuse mit warmem Wasser von etwa 50°C gewaschen und mit Essig- bzw. Ameisensäure abgesäuert.

Man erhält eine ausgezeichnete Grünfärbung, die sehr gute Schweiß-, Wasch- und Wasserechtheit besitzt und keinen Grauschleier aufweist.

### BEISPIEL 11

15 Teile des Farbstoffes C.I. 18170 (3. Auflage, Band 4) werden mit 600 Teilen heißem, weichem Wasser von 80°C übergossen; anschließend werden 10 Teile der Verbindung des Beispiels 3 hinzugefügt. Die Lösung wird unter kräftigem Rühren zu 200 Teilen einer 4%igen wäßrigen Zubereitung eines handelsüblichen Johannisbrotkernmehlderivates hinzugegeben. Die Lösung wird danach auf etwa 50°C abekühlt und mit einem Gemisch aus 20 Teilen Chromfluorid, 30 Teilen Ameisensäure (85%) und 100 Teilen heißem Wasser versetzt. Abschließend wird die Farbstoffzubereitung mit kaltem, weichem Wasser auf 1000 Teile aufgefüllt.

Mit der auf diese Weise erhaltenen Farbflotte wird ein Wollkammzug getränkt und zwischen zwei Foulardwalzen abgequetscht, wobei die Gewichtszunahme ca. 100% beträgt, und anschließend 40 Min. in einem Dämpfer mit Sattdampf bei 102—103°C kontinuierlich gedämpft. Danach wird die Färbung mit warmem Wasser von 50°C abermals gespült. Man erhält eine ausgezeichnete Graufärbung mit sehr guter Schweiß-, Walk-, Wasch- und Heißwasserechtheit.

### BEISPIEL 12

Man verfährt wie im Beispiel 11 angegeben, jedoch mit dem Unterschied, daß man anstelle der dort angeführten Verbindung 10 Teile der Verbindung des Beispiels 7 einsetzt.

Auch hierdurch erhält man eine Graufärbung, die keinen Grauschleier hat.

### BEISPIEL 13

10 Teile des 1:2-Chromkomplexes des Farbstoffes 2-Aminophenol-4-äthylsulfon → 1-Methylsulfonylamino-7-oxynaphthalin werden in 600 Teilen heißem, weichem Wasser von 80°C gelöst. anschließend wird ein Gemisch aus 8 Teilen der Verbindung des Beispiels 2 und 2 Teilen der Verbindung folgender Formel

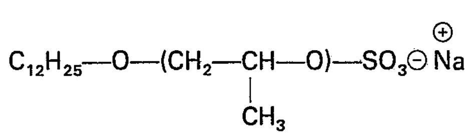

und 2 Teilen Diäthylenglykolmonobutyläther hinzugegeben. Die Lösung wird unter ständigem Rühren zu 200 Teilen einer 4%igen wäßrigen Zubereitung eines handelsüblichen Johannisbrotkernmehlderivates hinzugefügt. Darauf wird die homogene Lösung mit 20 Teilen Essigsäure (60%) versetzt. Anschließend wird die Farbstoffzubereitung mit kaltem, weichem Wasser auf 1000 Teile aufgefüllt.

Ein Kammzug aus Polyamid 6-Fasern wird mit dieser Farbflotte foulardiert, wobei die Flottenaufnahme etwa 80% beträgt, und anschließend in einem Dämpfer bei 102—103°C während 15 Min. kontinuierlich gedämpft. Danach wird der Kammzug auf einer Lisseuse mit warmem Wasser von 50°C gewaschen und gespült. Man erhält eine sehr gleichmäßige Graufärbung mit sehr guter Licht-, Wasch- und Schweißechtheit.

BEISPIEL 14

Eine Mischung aus 20 Teilen des Farbstoffes gemäß C.I. 15710 (3. Auflage, Band 4) und 500 Teilen weichem Wasser von etwa 90°C wird unter Rühren mit 10 Teilen der Verbindung des Beispiels 2 und 3 Teilen Natriumlaurylsulfonat und 2 Teilen Benzylalkohol versetzt und dann zu 100 Teilen einer 4%igen wäßrigen Zubereitung eines handelsüblichen Johannisbrotkernmehlderivates hinzugegeben. Darauf wird die Lösung auf etwa 50°C abgekühlt und mit einem Gemisch aus 12 Teilen Chromfluorid, 20 Teilen Ameisensäure (85%) und 100 Teilen heißem Wasser versetzt. Abschließend wird die Farbstoffzubereitung mit kaltem, weichem Wasser auf 1000 Teile aufgefüllt.

Mit dieser Farbpaste wird ein Wollkammzug auf einer Vigoureux-Druckmaschine (Walze 50:50) bedruckt und anschließend 20 Min. in einem Druckdämpfer mit Sattdampf bei etwa 108°C gedämpft. Danach wird der Kammzug auf einer Lisseuse mit warmem Wasser von 50°C gewaschen, gespült und mit Ameisensäure abgesäuert.

Man erhält auf diese Weise einen Schwarzdruck mit sehr guten Licht- und Naßechtheiten.

BEISPIEL 15

5 Teile des 1:2-Chromkomplexes des Monoazofarbstoffes Anthranilsäure → 1-Phenyl-3-methylpyrazolon werden mit 500 Teilen weichem Wasser von 70°C übergossen; anschließend wird ein Gemisch aus 4 Teilen der Verbindung des Beispiels 5 und 2 Teile Natriumlaurylsulfonat und 2 Teile der Verbindung folgender Formel

$$C_{12}H_{25}—SO_2—NH—CH_2—CH_2—OH$$

hinzugefügt. Die Mischung wird auf 80—90°C erhitzt und unter kräftigem Rühren zu 100 Teilen einer 4%igen wäßrigen Zubereitung eines handelsüblichen Johannisbrotkernmehlderivates hinzugegeben. Darauf wird die Lösung unter Rühren mit 5 Teilen Essigsäure (60%) versetzt. Die nunmehr entstandene Farbflotte wird dann mit weichem Wasser auf 1000 Teile aufgefüllt. Diese Farbflotte wird mittels eines Auftragswerkes auf einen Polyamid 6.6-Tufted-Teppich gleichmäßig aufgetragen, wobei die Gewichtszunahme ca. 300% beträgt, und anschließend wird der Teppich 15 Minuten in einem Dämpfer mit Sattdampf bei 102—103°C kontinuierlich gedämpft. Danach wird er auf einer Kontinue-Breitwaschmaschine mit warmem Wasser von 50°C gewaschen und gespült.

Man erhält eine sehr gleichmäßige Gelbfärbung, die keinen Grauschleier enthält und sehr gute Licht-, Wasser- und Shampooechtheit aufweist.

BEISPIEL 16

10 Teile des Farbstoffes folgender Formel

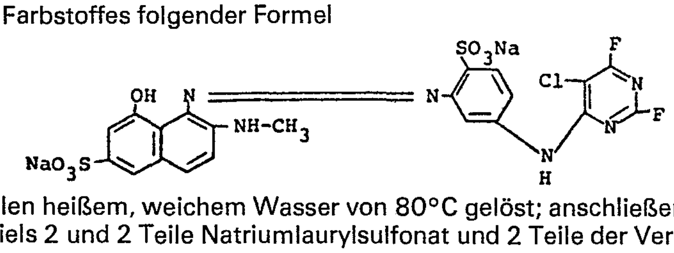

werden in 600 Teilen heißem, weichem Wasser von 80°C gelöst; anschließend werden 8 Teile der Verbindung des Beispiels 2 und 2 Teile Natriumlaurylsulfonat und 2 Teile der Verbindung folgender Formel

$$C_{12}H_{25}—SO_2—NH—CH_2—CH_2—O—CH_2—CH_2—CN$$

hinzugefügt. Die Lösung wird unter ständigem Rühren zu 200 Teilen einer 4%igen wäßrigen Zubereitung eines handelsüblichen Johannisbrotkernmehlderivates hinzugegeben. Hierauf wird die homogene Lösung mit 5 Teilen Essigsäure (60%) versetzt. Abschließend wird die Farbstoffzubereitung mit kaltem, weichem Wasser auf 1000 Teile aufgefüllt. Eine Wollgabardine wird mit dieser Farbflotte auf einem Foulard imprägniert; die Gewichtszunahme beträgt etwa 80%. Anschließend wird sie in einem Dämpfer mit Sattdampf während 15 Min. bei 102—103°C kontinuierlich gedämpft. Danach wird die Färbung mit warmem Wasser von 50°C, das einen pH-Wert von etwa 8,5 aufweist, gewaschen, gespült und mit Essig- bzw. Ameisensäure abgesäuert.

Man erhält eine brillante Rotfärbung, die keinen Grauschleier enthält.

BEISPIEL 17

20 Teile des Farbstoffes gemäß C.I. 58005 (3. Auflage, Band 4) werden in 600 Teilen heißem, weichem Wasser von 80°C gelöst; anschließend wird ein Gemisch aus 10 Teilen der Verbindung des Beispiels 1 und 4 Teilen Natriumlaurylsulfonat und 2 Teilen Äthylenglykolmonobutyläther hinzugegeben. Die Lösung wird unter kräftigem Rühren zu 200 Teilen einer 4%igen wäßrigen Zubereitung eines handelsüblichen Johannisbrotkernmehlderivates hinzugefügt. Die Lösung wird danach auf etwa 50°C abgekühlt und mit einem Gemisch aus 20 Teilen Chromfluorid, 30 Teilen Ameisensäure (85%) und 100 Teilen heißem Wasser versetzt. Abschließend wird die Farbstoffzubereitung mit kaltem, weichem Wasser auf 1000 Teile aufgefüllt.

Mit den auf diese Weise erhaltenen Farbstoffen wird lose Wolle getränkt und zwischen zwei Foulardwalzen abgequetscht, wobei die Gewichtszunahme ca. 120% beträgt, und anschließend 45 Min. in einem Dämpfer mit Sattdampf bei 102—103°C kontinuierlich gedämpft. Danach wird die Färbung mit warmem Wasser von 50°C abermals gespült.

Man erhält eine ausgezeichnete blaustichige Rotfärbung mit sehr guter Schweiß-, Wasser- und Waschechtheit.

BEISPIEL 18

15 Teile des Dispersionsfarbstoffes folgender Formel

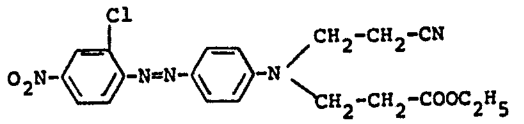

werden unter kräftigem Rühren in 500 Teile warmem Wasser von 50°C, das 12 Teile der Verbindung des Beispiels 7 enthält, eingestreut, Die Dispersion wird unter ständigem Rühren zu 200 Teilen einer 4%igen wäßrigen Zubereitung eines handelsüblichen Johannisbrotkernmehlderivates hinzugegeben. Darauf wird die Farbstoffzubereitung unter Rühren mit 5 Teilen Essigsäure (60%) versetzt. Abschließend wird sie mit kaltem Wasser auf 1000 Teile aufgefüllt.

Mit dieser Farbflotte wird ein Gewebe aus Polyäthylenterephthalat auf einem Foulard imprägniert, wobei die Flottenaufnahme ca. 60% beträgt, und 5 Min. in einem Druckdämpfer bei 135°C gedämpft. Danach wird die Färbung mit warmem Wasser von ca. 50°C abermals gespült.

Man erhält auf diese Weise eine sehr gleichmäßige Scharlachfärbung.

BEISPIEL 19

10 Teile des Farbstoffes C.I. 48040 (3. Auflage, Band 4) werden in 350 Teilen heißem, weichem Wasser von 90°C, das 1 Teil Essigsäure (60%) enthält, gelöst.

Und 6 Teile des Farbstoffes C.I. 40215 (3. Auflage, Band 4) werden in 350 Teilen heißem, weichem Wasser von 90°C gelöst. Zunächst die letztgenannte Farbstofflösung und anschließend die Lösung des kationischen Farbstoffes werden unter Rühren zu 75 Teilen einer 4%igen wäßrigen Zubereitung eines handelsüblichen Johannisbrotkernmehlderivates, die 10 Teile der Verbindung des Beispiels 2 enthält, hinzugegeben. Abschließend wird die Farbstoffzubereitung mit kaltem, weichem Wasser auf 1000 Teile aufgefüllt.

Ein Möbelbezugsstoff, bestehend aus Polyacrylnitrilfasern (Pol) und Baumwolle (Rücken), wird auf einem Foulard mit der oben beschreibenen Farbflotte imprägniert, wobei die Flottenaufnahme ca. 100% beträgt. Anschließend wird er in einem Dämpfer während 15 Min. bei 102—103°C kontinuierlich gedämpft. Danach wird die Färbung auf einer Kontinue-Breitwaschmaschine mit warmem Wasser von 50°C abermals gespült.

Man erhält eine hervorragende Gelbfärbung, die keinen Grauschleier aufweist.

BEISPIEL 20

8 Teile des Farbstoffes C.I. 51004 (3. Auflage, Band 4) werden in 350 Teilen heißem, weichem Wasser von 90°C, das 1 Teil Essigsäure (60%) enthält, gelöst.

Und 6 Teile des Farbstoffes folgender Formel

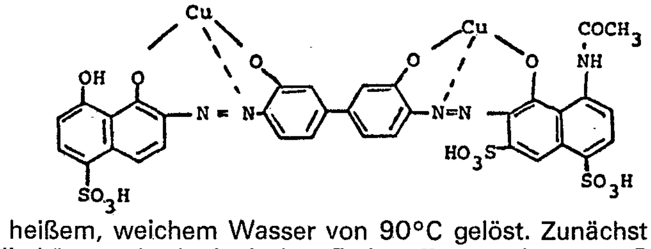

werden in 350 Teilen heißem, weichem Wasser von 90°C gelöst. Zunächst die letztgenannte Farbstofflösung und dann die Lösung des kationischen Farbstoffes werden unter Rühren zu 75 Teilen einer 4%igen wäßrigen Zubereitung eines handelsüblichen Johannisbrotkernmehlderivates, die 7 Teile der Verbindung des Beispiels 1 und 3 Teile Natriumlaurylsulfonat und 2 Teile Triacetin enthält, hinzugegeben. Abschließend wird die Farbstoffzubereitung mit kaltem, weichem Wasser auf 1000 Teile aufgefüllt.

Ein Möbelplüsch, bestehend aus Polyacrylnitrilfasern (Pol) und Baumwolle (Rücken), wird auf einem Foulard mit der oben erwähnten Farbflotte imprägniert, wobei die Flottenaufnahme ca. 100% beträgt. Anschließend wird er in einem Dämpfer während 15 Min. bei 102—103°C kontinuierlich gedämpft. Danach wird die Färbung auf einer Kontinue-Breitwaschmaschine mit warmem Wasser von 50°C abermals gespült. Man erhält eine ausgezeichnete Blaufärbung, die keinen Grauschleier aufweist.

## Patentansprüche

1. Oberflächenaktive Ester von Acylaminocarbonsäuren und Polyolen, dadurch gekennzeichnet, daß sie sich von Acylaminocarbonsäuren der Formel

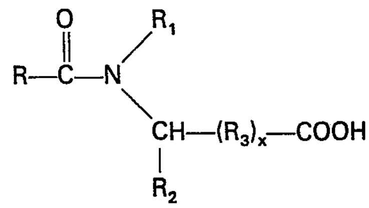

in der

R für Wasserstoff, $C_1$—$C_4$-Alkyl, $C_1$—$C_{22}$-Alkoxy, $C_3$—$C_{22}$-Alkenyloxy, Phenyl, Naphthyl, Benzyl, Phenylethyl, Naphthylmethyl, Phenyloxy, Benzyloxy, Phenylethyloxy oder für einen Rest

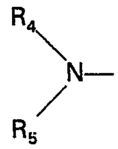 steht,

$R_1$, $R_4$ und $R_5$ für Wasserstoff, $C_1$—$C_{22}$-Alkyl, $C_3$—$C_{22}$-Alkenyl, Cyclohexyl, Tetrahydronaphthyl, Dekahydronaphthyl, Abietyl, Phenyl, Naphthyl, Benzyl oder Phenylethyl stehen, oder

$R_5$ für einen Rest der Formel

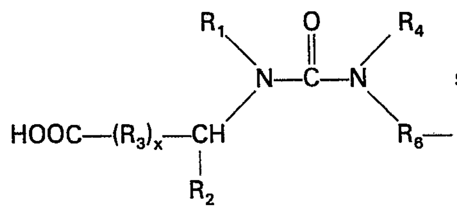 steht,

$R_6$ für $C_2$—$C_8$-Alkylen oder einen Rest der Formeln

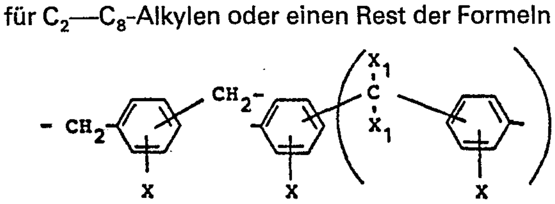 0 oder 1 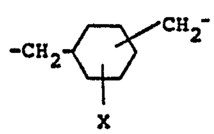

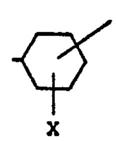 X, $X_1$ = Wasserstoff, $C_1$—$C_4$-Alkyl

$R_2$ für H, COOH, COO $C_1$—$C_4$-Alkyl oder $C_1$—$C_6$-Alkyl,
$R_3$ für $C_1$—$C_{10}$-Alkylen und
x für 0 oder 1 stehen

und in der mindestens einer der Reste $R_1$, $R_2$, $R_3$, $R_4$ und $R_5$ mindestens 6 Kohlenstoffatome enthält, und die Alkyl-, Alkenyl- und die cyclischen Reste durch Fluor, Chlor, Brom, Cyan, Carboxyl, $C_1$—$C_4$-Alkoxycarbonyl, Carbamoyl oder $C_1$—$C_4$-Alkoxy und die cyclischen Reste außerdem durch $C_1$—$C_4$-Alkyl substituiert sein können und von Glycerin, Erythrit, Erythrose, Pentosen oder Hexosen, Pentiten oder

Hexiten, Di- oder Oligosacchariden, Hydrolysate von Polysacchariden, Polysacchariden, Formosen, Polyvinylalkoholen, sowie Hydroxyalkylethern oder partiellen Estern oder Ethern von Polyhydroxylverbindungen ableiten.

2. Oberflächenaktive Ester gemäß Anspruch 1, dadurch gekennzeichnet, daß sie sich von Acylaminocarbonsäuren der Formel

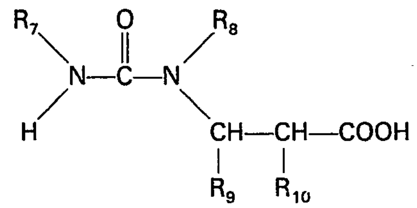

oder

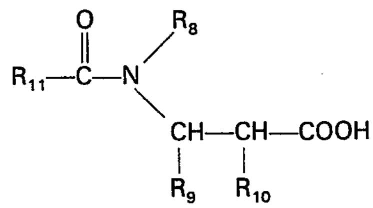

worin

$R_7$ für $C_1$—$C_{22}$-Alkyl, $C_3$—$C_{22}$-Alkenyl, Cyclohexyl, Tetrahydronaphthyl, Dekahydronaphthyl, Abietyl, Phenyl, Naphthyl, Benzyl, Phenylethyl oder einen Rest der Formel

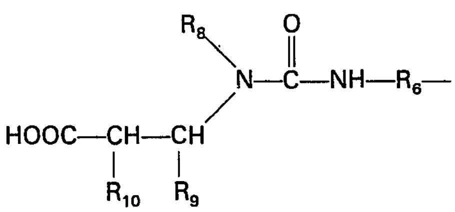

wobei

$R_6$ die in Anspruch 1 angegebene Bedeutung hat,
$R_8$ für Wasserstoff, $C_1$—$C_{22}$-Alkyl, $C_3$—$C_{22}$-Alkenyl, Cyclohexyl, Tetrahydronaphthyl, Dekahydronaphthyl, Abietyl, Benzyl oder Phenylethyl,
$R_9$ für Wasserstoff oder Carboxyl und
$R_{10}$ und $R_{11}$ für Wasserstoff oder Methyl stehen, mindestens einer der Reste $R_7$ oder $R_8$ mindestens 8 Kohlenstoffatome besitzt, und die Alkyl-, Alkenyl- und cyclischen Reste die in Anspruch 1 genannten Substituenten tragen können,
und mindestens 6 Kohlenstoffatome enthaltende Polyolen, ableiten.

3. Oberflächenaktive Ester gemäß Anspruch 1, dadurch gekennzeichnet, daß sie sich von Sorbit, Glucose, Alkyl- oder Hydroxyalkylglycosiden, Tetralose, Raffinose, Saccharose oder deren Mischungen ableiten.

4. Verfahren zur Herstellung von oberflächenaktiven Estern des Anspruchs 1, dadurch gekennzeichnet, daß man Carbonsäuren der Formel

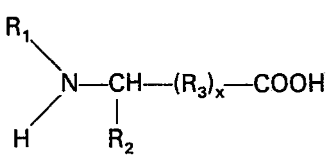

worin

$R_1$, $R_2$, $R_3$ und x die in Anspruch 1 angegebene Bedeutung haben,
ihre Alkylester, Anhydride oder Halogenide mit den in Anspruch 1 genannten Polyolen und Acylierungsmitteln der Formeln

$$R—CO—Y \quad R_4—NCO \text{ oder } OCN—R_6—NCO$$

worin

R, $R_4$ und $R_6$ die in Anspruch 1 angegebene Bedeutung haben, und

Y für eine $C_1$—$C_4$-Alkoxygruppe, Halogen oder die Gruppe —O—COR steht,

in beliebiger Reihenfolge umsetzt.

5. Verfahren gemäß Anspruch 4, dadurch gekennzeichnet, daß pro Äquivalent Carboxylgruppe 0,8 bis 1,2 Mol aliphatisches Polyol eingesetzt werden.

6. Verwendung der Verbindungen gemäß Anspruch 1 als Hilfsmittel beim Färben und Bedrucken von Materialien aus natürlichen und/oder synthetischen Fasern.

7. Verwendung der Verbindungen gemäß Anspruch 1 als Hilfsmittel beim kontinuierlichen Färben und Bedrucken von Textilmaterialien aus natürlichen und/oder synthetischen Fasern.

**Revendications**

1. Esters tensioactifs d'acides acylaminocarboxyliques et de polyols, caractérisés en ce qu'ils dérivent d'acides acylaminocarboxyliques de formule

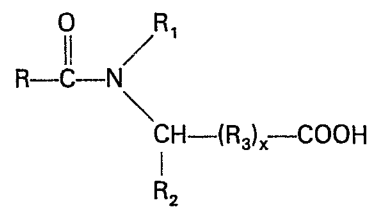

dans laquelle

R représente l'hydrogène, un groupe alkyle en $C_1$—$C_4$, alcoxy en $C_1$—$C_{22}$, alcényloxy en $C_3$—$C_{22}$, phényle, naphtyle, benzyle, phényléthyle, naphtylméthyle, phényloxy, benzyloxy, phényléthyloxy, ou un reste

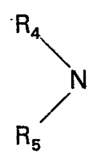

$R_1$, $R_4$ et $R_5$ représentent des atomes d'hydrogène, des groupes alkyle en $C_1$—$C_{22}$, alcényle en $C_3$—$C_{22}$, cyclohexyle, tétrahydronaphtyle, décahydronaphtyle, abiétyle, phényle, naphtyle, benzyle ou phényléthyle, ou bien

$R_5$ représente un reste de formule

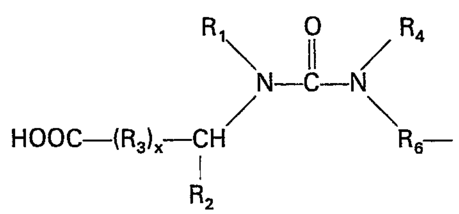

$R_6$ représente un groupe alkylène en $C_2$—$C_8$ ou un reste répondant à l'une des formules

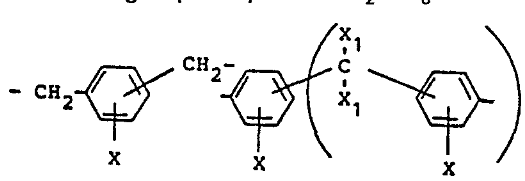 o ou 1 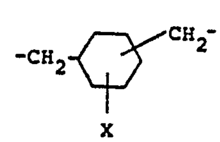

X et $X_1$ représente des atomes d'hydrogène ou des groupes alkyle en $C_1$—$C_4$,

$R_2$ représente H, COOH, COOalkyle en $C_1$—$C_4$ ou alkyle en $C_1$—$C_6$,

$R_3$ représente un groupe alkylène en $C_1$—$C_{10}$, et

x est égal à 0 ou 1,

l'un au moins des restes $R_1$, $R_2$, $R_3$, $R_4$ et $R_5$ contenant au moins 6 atomes de carbone, et les restes alkyle, alcényle et les restes cycliques pouvant être substitués par le fluor, le chlore, le brome, des groupes cyano, carboxyle, (alcoxy en $C_1$—$C_4$)-carbonyle, carbamoyle ou alcoxy en $C_1$—$C_4$, les restes cycliques pouvant en outre être substitués par des groupes alkyle en $C_1$—$C_4$, et du glycérol, de l'érythritol,

de l'érythrose, de pentoses ou d'hexoses, de pentites ou d'hexites, de di- ou oligo-saccharides, d'hydrolysats de polysaccharides, de polysaccharides, de formoses, d'alcohols polyvinyliques ou encore d'ethers hydroxyalkyliques ou d'esters ou éthers partiels de composés polyhydroxylés.

2. Esters tensioactifs selon la revendication 1, caractérisés en ce qu'ils dérivent d'acides acylaminocarboxyliques de formules

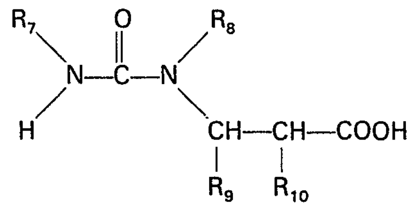

ou

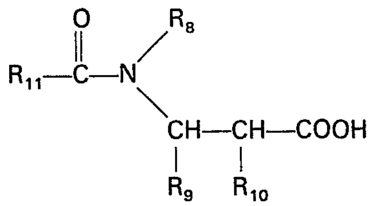

dans lesquelles

$R_7$ représente un groupe alkyle en $C_1$—$C_{22}$, alcényle en $C_3$—$C_{22}$, cyclohexyle, tétrahydronaphtyle, décahydronaphtyle, abiétyle, phényle, naphtyle, benzyle, phényléthyle, ou un reste de formule

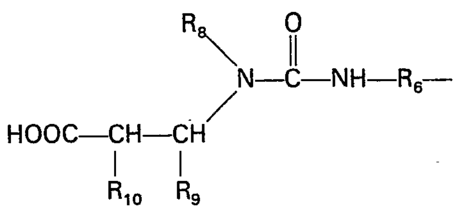

dans laquelle

$R_6$ a les significations indiquées dans la revendication 1,

$R_8$ représente l'hydrogène, un groupe alkyle en $C_1$—$C_{22}$, alcényle en $C_3$—$C_{22}$, cyclohexyle, tétrahydronaphtyle, décahydronaphtyle, abiétyle, benzyle ou phényléthyle,

$R_9$ représente l'hydrogène ou un groupe carboxyle, et

$R_{10}$ et $R_{11}$ représentent des atomes d'hydrogène ou des groupes méthyle, l'un au moins des restes $R_7$ et $R_8$ contenant au moins 8 atomes de carbone, et les restes alkyle, alcényle et les restes cycliques pouvant porter les substituants mentionnés dans la revendication 1, et de polyols contenant au moins 6 atomes de carbone.

3. Esters tensioactifs selon la revendication 1, caractérisés en ce qu'ils dérivent du sorbitol, du glucose, d'alkyl- ou hydroxyalkyl-glucosides, du tétralose, du raffinose, du saccharose ou de leurs mélanges.

4. Procédé pour la préparation des esters tensioactifs de la revendication 1, caractérisé en ce que l'on fait réagir dans un ordre quelconque des acides carboxyliques de formule

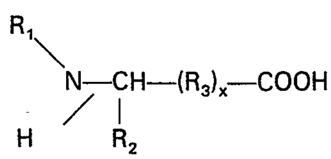

dans laquelle

$R_1$, $R_2$, $R_3$ et x ont les significations indiquées dans la revendication 1,

leurs esters alkyliques, anhydrides ou halogénures, avec les polyols mentionnés dans la revendication 1 et des agents acylants répondant aux formules

$$R—CO—Y \quad R_4—NCO \text{ ou } OCN—R_6—NCO$$

dans lesquelles

R, $R_4$ et $R_6$ ont les significations indiquées dans la revendication 1, et

Y représente un groupe alcoxy en $C_1$—$C_4$, un halogène ou le groupe —O—COR.

5. Procédé selon la revendication 4, caractérisé en ce que l'on utilise de 0,8 à 1,2 moles de polyol aliphatique par équivalent de groupe carboxyle.

6. Utilisation des composés selon la revendication 1, en tant que produits auxiliaires dans la teinture et l'impression de matières en fibres naturelles et/ou synthétiques.

7. Utilisation des composés selon la revendication 1, en tant que produits auxiliaires dans la teinture et l'impression continue de matières textiles en fibres naturelles et/ou synthétiques.

**Claims**

1. Surface-active esters of acylaminocarboxylic acids and polyols, characterised in that they are derived from acylamino-carboxylic acids of the formula

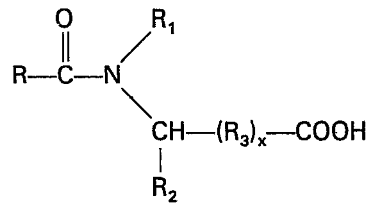

in which

R represents hydrogen, $C_1$—$C_4$-alkyl, $C_1$—$C_{22}$-alkoxy, $C_3$—$C_{22}$-alkenoxy, phenyl, naphthyl, benzyl, phenylethyl, naphthylmethyl, phenyloxy, benzyloxy, phenylethyloxy or a radical

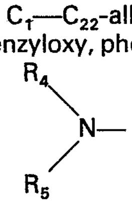

$R_1$, $R_4$ and $R_5$ represent hydrogen, $C_1$—$C_{22}$-alkyl, $C_3$—$C_{22}$-alkenyl, cyclohexyl, tetrahydronaphthyl, decahydronaphthyl, abietyl, phenyl, naphthyl, benzyl or phenylethyl or

$R_5$ represents a radical of the formula

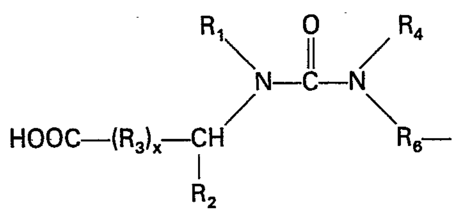

$R_6$ represents $C_2$—$C_8$-alkylene or a radical of the formulae

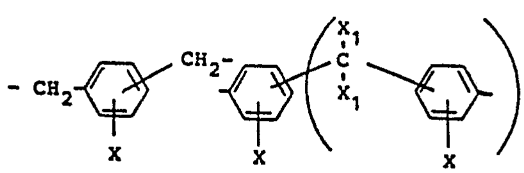 o or 1 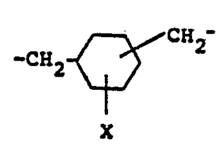 and

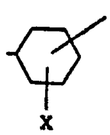 X and $X_1$ = hydrogen or $C_1$—$C_4$-alkyl

$R_2$ represents H, COOH, COO $C_1$—$C_4$-alkyl or $C_1$—$C_6$-alkyl,

$R_3$ represents $C_1$—$C_{10}$-alkylene and

x represents 0 or 1,

and in which at least one of the radicals $R_1$, $R_2$, $R_3$, $R_4$ and $R_5$ contains at least 6 carbon atoms, and the alkyl, alkenyl and cyclic radicals can be substituted by fluorine, chlorine, bromine, cyano, carboxyl, $C_1$—$C_4$-alkoxycarbonyl, carbamoyl or $C_1$—$C_4$-alkoxy and the cyclic radicals can also be substituted by $C_1$—$C_4$-alkyl, and from glycerine, erythritol, erythrose, pentoses or hexoses, pentites or hexites, di- or oligo-saccharides, hydrolysates of polysaccharides, polysaccharides, formoses, polyvinyl alcohols, as well as from hydroxyalkyl ethers or partial esters or ethers of polyhydroxyl compounds.

2. Surface-active esters according to Claim 1, characterised in that they are derived from acylaminocarboxylic acids of the formula

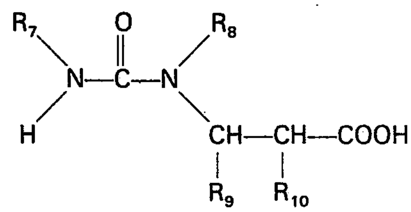

or

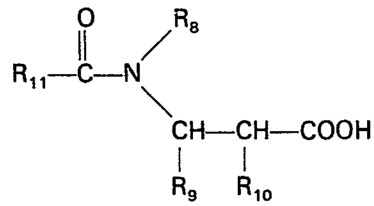

in which

$R_7$ represents $C_1$—$C_{22}$-alkyl, $C_3$—$C_{22}$-alkenyl, cyclohexyl, tetrahydronaphthyl, decahydronaphthyl, abietyl, phenyl, naphthyl, benzyl, phenylethyl or a radical of the formula

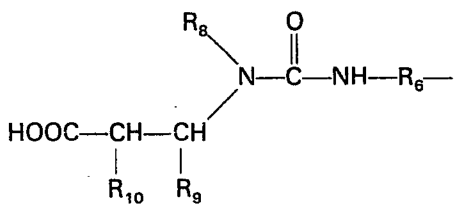

wherein

$R_6$ has the meaning given in Claim 1,

$R_8$ represents hydrogen, $C_1$—$C_{22}$-alkyl, $C_3$—$C_{22}$-alkenyl, cyclohexyl, tetrahydronaphthyl, decahydronaphthyl, abietyl, benzyl or phenylethyl,

$R_9$ represents hydrogen or carboxyl and

$R_{10}$ and $R_{11}$ represent hydrogen or methyl, at least one of the radicals $R_7$ or $R_8$ has at least 8 carbon atoms and the alkyl, alkenyl and cyclic radicals can carry the substituents mentioned in Claim 1, and from polyols containing at least 6 carbon atoms.

3. Surface-active esters according to Claim 1, characterised in that they are derived from sorbitol, glucose, alkyl or hydroxyalkyl glycosides, tetralose, raffinose, saccharose or mixtures thereof.

4. Process for the preparation of surface-active esters of Claim 1, characterised in that carboxylic acids of the formula

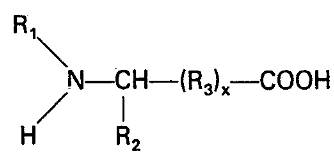

in which

$R_1$, $R_2$, $R_3$ and x have the meaning indicated in Claim 1, their alkyl esters, anhydrides or halides are reacted with the polyols mentioned in Claim 1 and acylating agents of the formulae

$$R—CO—Y \quad R_4—NCO \text{ or } OCN—R_6—NCO$$

wherein

R, $R_4$ and $R_6$ have the meaning indicated in Claim 1 and

Y represents a $C_1$—$C_4$-alkoxy group, halogen or the group —O—COR,

in any desired sequence.

5. Process according to Claim 4, characterised in that 0.8 to 1.2 mols of aliphatic polyol are employed per equivalent of carboxyl group.

6. Use of the compounds according to Claim 1 as auxiliaries in the dyeing and printing of materials made of natural and/or synthetic fibres.

7. Use of the compounds according to Claim 1 as auxiliaries for the continuous dyeing or printing of textile materials made of natural and/or synthetic fibres.